(19) **Europäisches Patentamt European Patent Office Office européen des brevets**

(11) **EP 3 681 448 B1**

(12) **EUROPEAN PATENT SPECIFICATION**

(45) Date of publication and mention of the grant of the patent:
**01.11.2023 Bulletin 2023/44**

(21) Application number: **18780237.6**

(22) Date of filing: **11.09.2018**

(51) International Patent Classification (IPC):
**A61F 9/00** *(2006.01)*    **A61K 9/00** *(2006.01)*
**A61K 47/02** *(2006.01)*    **A61P 27/00** *(2006.01)*

(52) Cooperative Patent Classification (CPC):
**A61F 9/0017; A61K 9/0051; A61K 47/02;**
**A61P 27/00;** A61F 2210/0004; A61F 2250/0068

(86) International application number:
**PCT/IB2018/056907**

(87) International publication number:
**WO 2019/053584 (21.03.2019 Gazette 2019/12)**

(54) **DEVICE FOR INTRAOCULAR RELEASE OF A MEDICAMENT**

VORRICHTUNG ZUR INTRAOKULAREN FREISETZUNG EINES ARZNEIMITTELS

DISPOSITIF D'ADMINISTRATION INTRAOCULAIRE DE MÉDICAMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2017 IT 201700101582**

(43) Date of publication of application:
**22.07.2020 Bulletin 2020/30**

(73) Proprietor: **Politecnico di Milano**
**20133 Milano (IT)**

(72) Inventors:
• **BOSCHETTI, Federica**
**20133 Milano (IT)**
• **FERRONI, Marco**
**20133 Milano (IT)**
• **CEREDA, Matteo Giuseppe**
**20133 Milano (IT)**

(74) Representative: **Rigamonti, Dorotea et al**
**Marchi & Partners S.r.l.**
**Via Vittor Pisani, 13**
**20124 Milano (IT)**

(56) References cited:
**US-A1- 2006 110 429    US-A1- 2011 054 408**
**US-A1- 2011 159 073    US-A1- 2016 045 363**

• **LIN MAO ET AL: "Enhanced Bioactivity of Mg-Nd-Zn-Zr Alloy Achieved with Nanoscale MgF 2 Surface for Vascular Stent Application", ACS APPLIED MATERIALS & INTERFACES, vol. 7, no. 9, 27 February 2015 (2015-02-27), pages 5320-5330, XP055530432, US ISSN: 1944-8244, DOI: 10.1021/am5086885**
• **LIN MAO ET AL: "A promising biodegradable magnesium alloy suitable for clinical vascular stent application", SCIENTIFIC REPORTS, vol. 7, no. 1, 11 April 2017 (2017-04-11), XP055530438, DOI: 10.1038/srep46343**

## Description

The present invention relates to a biodegradable device injectable in the posterior chamber of the eye, which comprises at least one active ingredient. The device allows a scheduled release of the active ingredient, where scheduled release means herein a release of predefined doses of said at least one active ingredient, that is a release in a quantum manner, in distinct and predefined moments over time.

Prior art

The term maculopathy refers to a series of diseases affecting the macula, the area at the center of the retina. Among these, the most common form is Age-Related Macular Degeneration (AMD). Other maculopathies found are diabetic macular edema, macular edema from retinal vein thrombosis, exudative myopic maculopathy, choroidal neovascularization (CNV) not from AMD.

AMD is the main cause of visual impairment among people over 50 years of age in developed countries. AMD is developed in two different forms, exudative and non-exudative. The exudative or neovascular form represents 90% of the cases of acute vision loss associated with AMD and is less frequent than the non-exudative form.

Vascular endothelial growth factors (VEGF) are potent angiogenic agents that trigger the formation of new blood vessels, or neovascularization of the choroid, whose presence is pathognomonic of exudative maculopathy.

Currently, the treatment of maculopathies is represented by intravitreal injections of drugs based on vascular endothelial growth factor inhibitors (anti-VEGF). Clinical studies show that anti-VEGF drugs, injected monthly, are capable of maintaining the initial visual ability in over 90% of cases and to induce an improvement in 25-40% of patients treated.

An essential feature for the effectiveness of the treatment is that the tissue is periodically exposed to the anti-VEGF drug at a predefined dose.

Among the known anti-VEGFs employed in maculopathies, Pegaptanib, Ranibizumab and Aflibercept are cited. The approved therapeutic protocol for Ranibizumab includes one administration per month until maximum visual acuity and/or the absence of neovascularization activity are achieved. This treatment is followed by frequent monitoring and treatment as needed. Aflibercept is injected into the affected eye once a month for three consecutive months, followed by a single injection every two months.

Treatments with repeated injections are difficult for patient compliance as well as significantly expensive.

US2006110429 describes controlled drug release ocular systems capable of releasing the drug with which they are loaded over a certain period of time. No release at doses is contemplated.

WO2011097634 describes an ocular implant for the two-step release of two doses of drug, where the implant is activated by a laser so as to break and allow release at the set time.

Also the implant described in WO2009097468 controls the release from the outside, the activation of the system is in fact obtained with a light source.

US2005244465 describes an ocular implant having a sandwich structure, where the central portion comprises the drug and the two outer layers are made of polymeric material. The shape parameters of said sandwich structure, together with the properties of the polymeric material, regulate the release of the drug over time. The highlighted solution has the drawback of the production in biodegradable polymeric material which involves the release of undesirable degradation products at the ocular level.

US2016045363 describes drug delivery devices for the controlled administration of a drug to an interior portion of the eye. In an embodiment, a "tube within a tube" design is disclosed, with an ocular fluid flow lumen.

A drug delivery system is disclosed in US2011054408 comprising at least two compartments loaded with one or more active ingredients.

An object of the present invention is a scheduled release device which solves the yet unresolved problems of the prior art devices with which physicians and patients have to cope today.

Description of the invention

The invention is defined by the independent claims. The device object of the present invention is an implantable device consisting of layers of biodegradable material, wherein a dose of at least one active ingredient is placed between the different layers, which is released in a quantum manner at predefined intervals.

Description of the figures

Figure 1: an embodiment of the implantable device according to the present invention.

Figure 2: a further embodiment of the implantable device according to the present invention.

Figure 3: a further embodiment of the implantable device according to the present invention.

Figure 4: representative photographs of a prototype according to the present invention, A) viewed from above, B) side view for the evaluation of the external diameters and C) lateral view and view of the closing disks.

Figure 5: 24 hour time-scale analysis of sample morphology: localizations are present before testing them (t = 0). After 24 hours, the flow of the fluid oxidized the magnesium localizations and then eroded the corrosion products, making the eroded surface flat.

Figure 6: Gaussian distribution of the typical local height after the corrosion test. The mean and standard deviation values of the normal distribution are shown for FISS equal to A) 0 Pa, B) 0.01 Pa and C) 0.032 Pa.

Figure 7: Trend over time of the average height decrease after 8, 24 and 48 hours of corrosion tests. In the first 8 hours there is no statistical difference between (i) FISS = 0.01 Pa and (ii) FISS = 0.032 Pa (one-way ANOVA test, $p < 0.05$).

Figure 8: Amount of decrease in height of Mg samples subjected to 24 hours of corrosion. A one-way ANOVA test ($p < 0.05$) did not show any statistical difference between static (0 Pa, white column) and the first case under dynamic conditions (0.01 Pa, black column). The second case of dynamic stimulation (0.032 Pa, gray column) is statistically different from both the static (0 Pa) and the first case of dynamic condition (0.01 Pa).

Figure 9: Trend curve (A) and histogram (B) of the corrosion rate of magnesium biodegradation as the FISS values change. The first derivative of the corrosion rate tends to go down to zero when the analysis switches from a static condition to the most stressful one analyzed (0.032 Pa).

Figure 10: Monthly biodegradation of a wall by applying a corrosion rate of 3.4 um/day (FISS = 0.032 Pa, left) or 1.9 um/day (FISS = 0 Pa, right).

Figure 11: Distribution of drugs on the equatorial plane of the vitreous cavity in different temporal phases after IVI administration of bevacizumab encapsulated within the device according to the present invention.

Figure 12: Distribution of the drug on different xy planes of the vitreous cavity: (a) 3D view, (b) z = 0.15 cm, (c) z = 0.30 cm, (d) z = 0.45 cm and (e) z = 0.60 cm from the equatorial plane (z = 0). All maps were exported after 30 seconds of simulation after IVI administration of bevacizumab encapsulated within the device.

Figure 13: Distribution of Bevacizumab in external vitreous (triangles), internal vitreous (rhombus) and total initial volume (circles). The line marked by the squares represents the total amount of bevacizumab during the simulation after its release from the device.

Figure 14: Development of the bevacizumab concentration in the RCS complex after 30 seconds of simulation.

Detailed description of the invention

The implantable device object of the present invention comprises at least two hollow solids inscribed one into the other, where said at least two hollow solids are made of biodegradable material. Said at least two hollow solids delimit: i) a volume, called core, inside the innermost of said hollow solids, and ii) one or more volumes, called crown, comprised between said innermost hollow solid and the hollow solid at the exterior thereof. Said core and at least said one crown are adapted to house drug doses.

The number of hollow solids inscribed one into the other that make up the implantable device determines the therapeutic regimen. By way of example, an implantable device consisting of two hollow solids inscribed one into the other, and therefore of two biodegradable layers, is capable, after implantation, of providing for the release of two drug doses. An implantable device consisting of three hollow solids inscribed one into the other is capable, after implantation, of providing for the release of three drug doses. Embodiments are provided which comprise up to twenty hollow solids inscribed one into the other, preferably up to ten, or up to five, preferably three hollow solids inscribed one into the other.

Said hollow solids are selected among the geometric solids. Preferably, they are polyhedra or rotation solids.

In one embodiment, the different hollow solids that make up the device according to the present invention are of a different type from each other; purely by way of example, in a device comprising two hollow solids, the innermost solid is a cylinder and the outer one is a parallelepiped.

In a further embodiment, the different hollow solids that make up the device according to the present invention are of the same type; purely by way of example, in a device comprising two hollow solids, the innermost solid is a prism and the outer one is a larger prism.

In a preferred embodiment, said hollow solids inscribed one into the other are concentric with each other.

In one embodiment, said hollow solids inscribed one into the other are arranged in a "matrioshka-like" structure, i.e. the innermost hollow solid is completely contained in the hollow solid at the exterior thereof.

**In** an alternative embodiment, said hollow solids inscribed one into the other are arranged so as to have an upper base and/or a lower base in common. That is, said hollow solids inscribed one into the other are, for example, cylinders, or parallelepipeds having different bases, that is, smaller ones moving from the outermost hollow solid to the innermost hollow solid, and the same height.

This "shared-base" embodiment is particularly preferred because it offers advantages from the constructive point of view. In fact, unlike the "matrioshka-like" structure, the embodiment in which the hollow solids inscribed one into the other have an upper base and/or a lower base in common allows the implementation of an empty device, in magnesium and/or magnesium alloy. Said device is then filled with the at least one active ingredient, in the desired formulation, and subsequently closed with said upper base and/or said lower base.

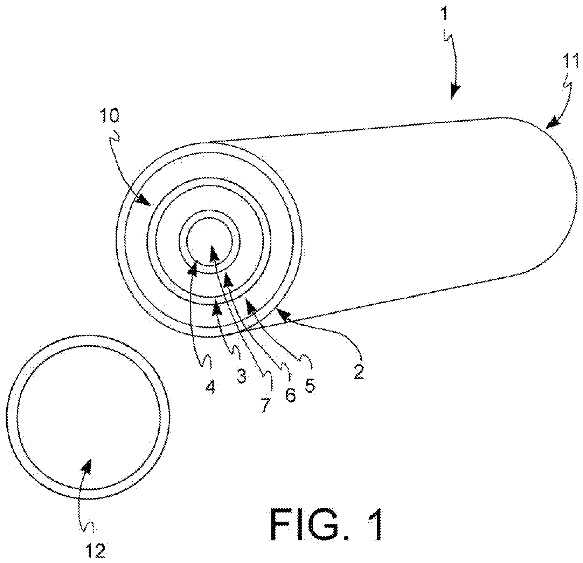

In the embodiment schematized in figure 1, provided by way of a non-limiting example of the scope of protection of the present invention, said device is cylindrical. Said cylindrical device 1 comprises three cylinders inscribed one into the other with the concentric bases, which identify two circular crowns. The lateral walls 2, 3, 4 of said cylinders are made of biodegradable material. Said three cylinders inscribed one into the other delimit one of the three volumes inside said device: a first crown 5, a second crown 6 and a core 7. Said first crown 5, said second crown 6 and said core 7 each comprise a drug dose. The dose of drug comprised in the first crown 5 is the first to be released from said device after implantation of said device 1 in the eye. In successive steps, the second dose of drug comprised in said second crown 6 is released and again the third dose of drug, comprised in said core 7. Said device 1 is closed at the upper end and at the lower end by an upper base 11 and a lower base 12. Said upper base 11 and said lower base 12 are made of biodegradable material.

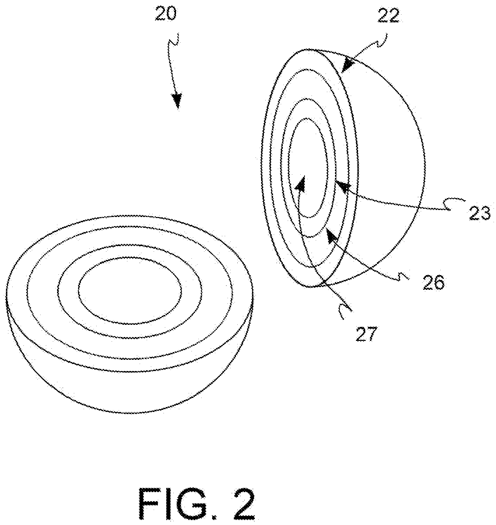

In an alternative embodiment, shown schematically in figure 2, said device is spherical and consists of two concentric hollow spheres, in the specific example an outer sphere 22 and an inner sphere 21, to determine a core 27 and a crown 26, where said core and said one crown each comprise a dose of drug. Said two concentric hollow spheres are made of biodegradable material according to the present invention.

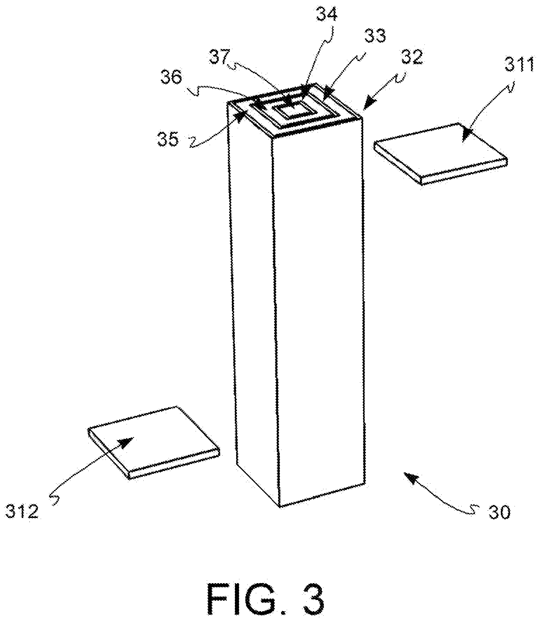

In the embodiment schematized in figure 3, said device is in the form of a parallelepiped, preferably with a square base. Said device 30 comprises three hollow parallelepipeds, inscribed one into the other. The lateral walls 32, 33, 34 of said parallelepipeds, respectively outer, middle and inner, are made of the biodegradable material according to the present invention. Said three hollow parallelepipeds delimit three volumes within said device 31: a first volume 35, a second volume 36 and a core 37. Said first volume 35, said second volume 36 and said core 37 each comprise a drug dose. The dose of drug comprised in the first volume 35 is the first to be released from said device after implantation of said device 31 in the eye. In successive steps, the second dose of drug, comprised in said second volume 36, and successively the third dose of drug, comprised in said core 37, are released. Said device 31 is closed at the ends by an upper base 311 and a lower base 312. Said upper base 311 and said lower base 312 are made of the biodegradable material according to the present invention.

The biodegradable material used in the device according to the present invention is a magnesium compound. The advantages found with the use of magnesium compounds in an implantable device are linked to a complete resorption, due to the poor corrosion resistance in humid environments and to a high biocompatibility of the corrosion products. Magnesium alloys such as aluminum alloys, lithium, calcium, zinc, manganese are particularly preferred embodiments.

In a preferred embodiment, said device is made of JDBM magnesium alloy, a Mg-2.5Nd-0.2Zn-0.4Zr alloy (wt%, JDBM) which possesses good mechanical properties and biocorrosion resistance. Alternatively, said alloy is JBDM-2, Mg-2.2Nd-0.1Zn-0.4Zr (wt%, referred to as JDMB-2).

The thickness of the magnesium layers determines the degradation rate thereof, where a greater thickness corresponds to a greater degradation time. In the embodiments according to the present invention providing an upper base and/or a lower base in common, where the degradation of said upper base and/or said lower base determines the opening towards the outside of each of the volumes comprised in said device, said upper base and said lower base are made of a thickness such as to guarantee the maintenance hereof up to the degradation of the innermost hollow solid.

The device according to the present invention is impermeable and becomes permeable as a function of the post-implantation time of said device. In a preferred embodiment, said permeability is obtained by dissolving the wall of the outermost hollow solid. Said dissolution, which takes place as a function of the intraocular residence time and the thickness thereof, leads to the release of the active ingredient contained in the outermost hollow solid. Thereafter, and following exposure of the wall of the inscribed hollow solid to the corrosion exerted by the intraocular fluid, there is a degradation of the wall of the hollow solid inscribed in said outermost hollow solid, with the release of the amount of active ingredient contained therein. In one embodiment, the wall thickness of said hollow solids is the same for each of said hollow solids. In an alternative embodiment, advantageously applied when said active ingredient is in liquid form, the thickness of the wall of said hollow solids varies, by increasing, going from said outermost hollow solid to said innermost hollow solid.

Said core, inside the innermost of said hollow solids and said one or more volumes, comprised between said innermost hollow solid and the hollow solid at the exterior thereof in one embodiment have the same volume. In an alternative embodiment, they have a different volume.

Said hollow solids inscribed one into the other, in one embodiment, are connected to each other by partitions, made of the same biodegradable material, where said partitions have, for example, a structural function.

Said at least one active ingredient with which said implantable device is loaded is an active ingredient selected from the active ingredients for intraocular use. Preferably, said active ingredient is a drug selected from the group comprising the anti-VEGF compounds, such as monoclonal antibodies: Ranibizumab, Bevacizumab, Brolucizumab; aptamers: Pegaptanib; fusion proteins: VEGF Trap Eye Aflibercept; Small-Interfering RNA (SiRNA); Anti-Platelet derived growth factor (PDGF); Anti Tyrosine Kinase or Tyrosine-kinase inhibitors; Anti receptor kinase; intracellular inhibitors of the tyrosine kinase cascade; kinase multi target inhibitors; mTOR inhibitors; integrin inhibitors; vascular disrupting agents (VGAs); anti-nicotine agents, anti-complement agents or complement inhibitors; immunomodulators; anti-oxidant agents; Ciliary neurotrophic factors (CNTF); corticosteroids; non-steroidal anti-inflammatory drugs; biological material, for example viral vectors including nucleotide sequences; cells, e.g. cells transfected with at least one of said viral vectors.

Said active ingredients are loaded individually or mixed.

In a preferred embodiment, said implantable device comprises three doses of an anti-VEGF compound, wherein said three doses each comprise the amount of active ingredient necessary for the treatment, where said quantities for each specific compound are known to those skilled in the art.

In one embodiment, said at least one active ingredient is loaded in liquid form, alternatively as a lyophilized product.

In one embodiment, the at least one active ingredient is loaded as such, alternatively with excipients known to those skilled in the art.

In one embodiment, the implantable device is cylindrical and has a height of about 10 mm, or about 5 mm and a base diameter of about 0.6 mm.

In one embodiment, the implantable device is spherical and the diameter is about 0.6 mm.

In an alternative embodiment, the implantable device is a parallelepiped with a square section, preferably with a height of about 10 mm, or about 5 mm and a diagonal at the base of about 0.6 mm.

A further aspect of the present invention is a method for producing an implantable device which comprises:

- making a structure of magnesium and/or magnesium alloys, where said structure comprises at least two hollow solids inscribed one into the other defining at least one innermost volume, called core, and at least one volume called crown, where said at least two volumes defined by said at least two hollow solids inscribed one into the other have both at least one opening towards the outside;
- loading of said structure with one or more active ingredients;
- closing said structure.

Said loading takes place by introducing in said at least two volumes obtained from said at least two hollow solids inscribed one into the other one or more active ingredients, in liquid or lyophilized form, as such or in formulation.

Said closure takes place with a closing element which is made of magnesium and/or magnesium alloy which closes said at least one opening towards the outside of each of said at least two volumes. In a preferred embodiment, shown in the embodiments in figure 1 and figure 3, said at least one opening towards the outside coincides with the upper base and/or the lower base of said hollow solids. Said closing element is therefore the upper base and/or the lower base of said outermost hollow solid.

Said closing element is made with such a thickness as to guarantee the maintenance thereof until the degradation of the innermost hollow solid in the device.

The implantable device object of the present invention is positioned in the posterior chamber of the eye, after being suspended in balanced saline. The degradation of the outermost hollow solid and, subsequently, of the innermost hollow solids, is determined by the corrosion induced by the movement of the vitreous on said hollow solids of biodegradable material.

In a further aspect, it is here described an intraocular treatment method which does not form part of the invention and comprises:

- providing a device comprising at least two hollow solids inscribed one into the other, where the walls of said at least two hollow solids are made of biodegradable material based on magnesium, and where said at least two hollow solids inscribed one into the other delimit a volume, called core, inside the innermost of said hollow solids and one or more volumes, called crowns, comprised between said innermost hollow solid and the hollow solid at the exterior thereof, where said volumes comprise at least one active ingredient;
- inserting said device into the posterior chamber of the eye; characterized in that said walls are impermeable continuous walls which become permeable as a function of the post-implantation time of said device, wherein preferably said walls become permeable by dissolution thereof.

Advantageously, the device according to the present invention does not require external actuators to cause the scheduled release of the drug contained therein.

The movement of the vitreous on said walls generates specific shear stress values ($\tau_{ij}$), where said shear stress

$$\tau_{ij} = \mu\left(\frac{\partial v_i}{x_j} + \frac{\partial v_j}{x_i}\right)$$

is calculated according to equation 1:

Said shear stress acts on the free surfaces of the device, defined (A) and is linearly related to the speed gradient that is created ( $\frac{\partial v_i}{x_j} + \frac{\partial v_j}{x_i}$ ), according to a factor determined by the dynamic viscosity of the fluid ($\mu$).

The phenomenon involves a progressive and homogeneous decrease in the thickness of the material ( $\frac{\Delta V}{A}$ ) which proceeds until complete erosion (dissolution), at substantially constant speed (CR) and in accordance with the

$$CR = \frac{\Delta V}{A \cdot t}$$

clinical treatment timing (t), as per equation 2:

When the erosion is complete, the drug comprised in the crown that is released spreads following the transport dynamics generated by the voluntary and involuntary eye movements, defined by the Navier-Stokes continuity equations, conservation of mass and Fick and related to the rheological properties of the vitreous humor, dynamic viscosity and density.

Once the first dose of drug has been distributed, the erosion of the hollow solid of said device starts and it becomes exposed. The process is reiterated as many times as the number of hollow solids inscribed one into the other that make up the implantable device, so as to satisfy diversified therapeutic regimes.

The following examples are purely exemplary and not limiting of the invention which is defined by the appended claims.

Example 1: Implementation of a prototype of the device according to the present invention.

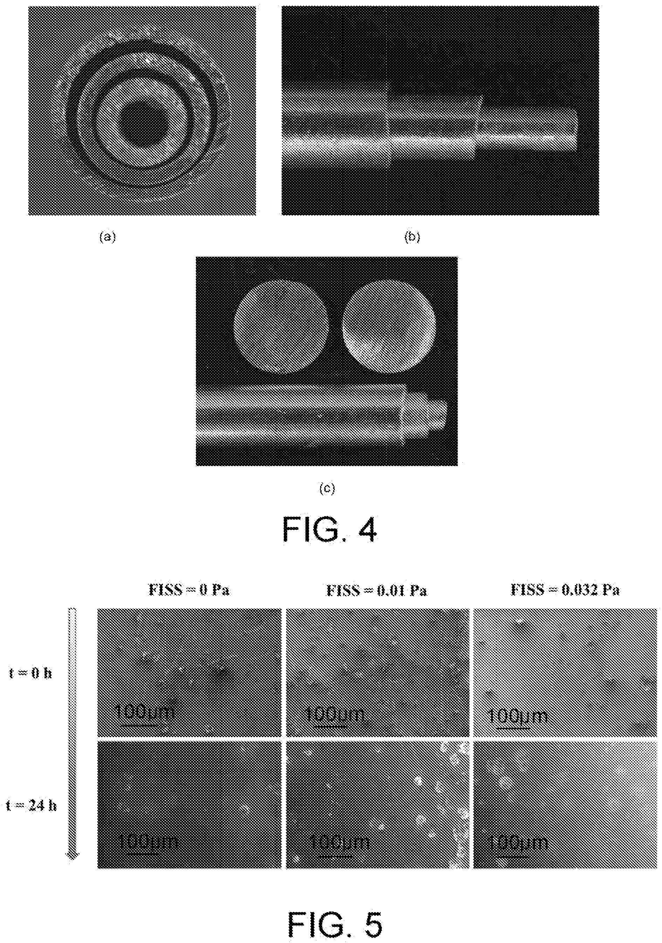

Three concentric mini-shells of different outer diameters and two closing disks were made according to the method described in Liu F et al. 2015, Mater Sci Eng C Mater Biol Appl. 48:400-7. Briefly, three JDBM alloy bars (Mg magnesium alloy comprising 2.5% wt Nd, 0.2% wt Zn, 0.4% wt Zr) were processed into empty billets and then extruded into unprocessed tubes at 350 °C. Their final size was 8.0 mm outer diameter and 0.5 mm thickness. The lamination of the semi-finished products of the extruded tube was then carried out at room temperature, with a reduction of the area between 10% and 15% at each passage. A further reduction of the area by means of lamination was carried out until thinner tubes with an outer diameter of 4.0 mm and a thickness of 250 um were obtained. As a final step, three microtubes with a thickness of 200 um were manufactured, the outer diameters of which were reduced to 2.0 mm, 1.5 mm and 1.0 mm respectively. Finally, each minitube was cut into 10 mini-shells of 20 mm in length. The insertion of the three mini-shells one inside the other was carried out, thus obtaining the configuration referred to in figures 4 A, B, C. A series of 3.0 mm diameter and 0.6 mm thickness disks were cut from cross sections of extruded and finished bars (Figure 4C). The prototype thus obtained is a 1:4 scale reproduction of the device according to the present invention. The larger dimensions of the prototype with respect to the dimensions of the device are useful for facilitating corrosion and release tests in vitro on an experimental model of the vitreous cavity in liquefied conditions, a 4 times enlarged reproduction of the real one (Stocchino et al. 2007 Phys MedBiol 52:2021-2034; Stocchino et al. 2010 Phys Med Biol 55:453-67.).

Example 2: Corrosion and release tests.

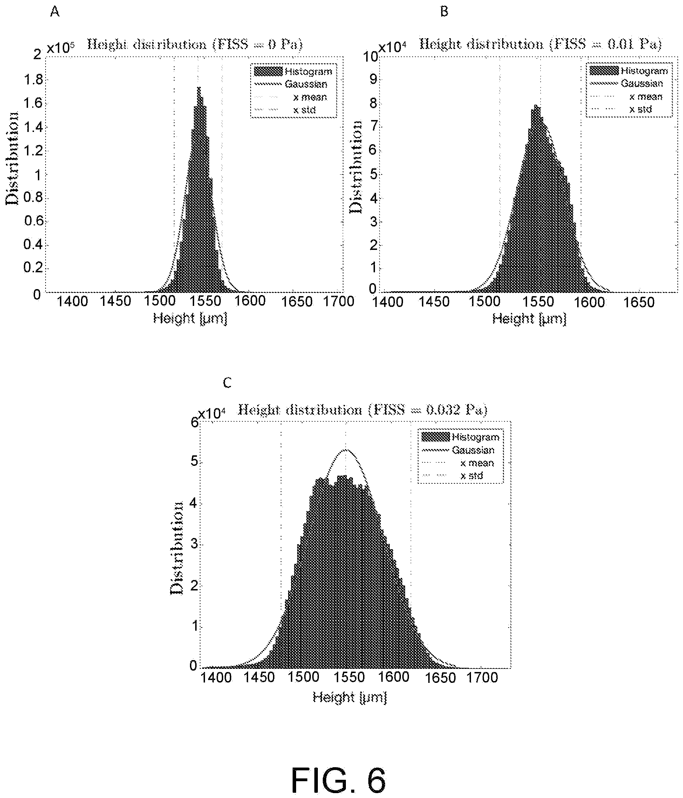

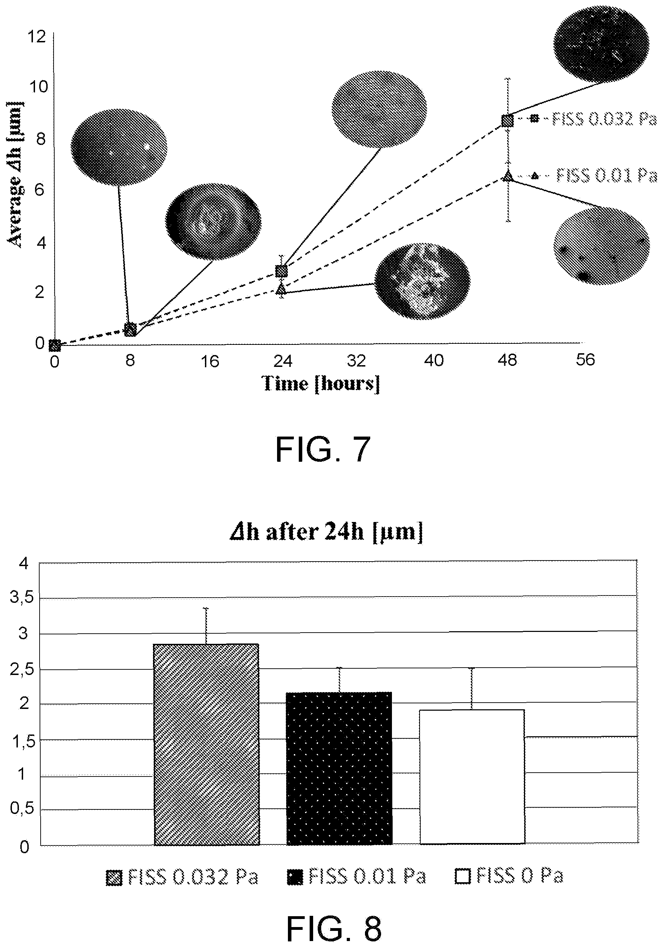

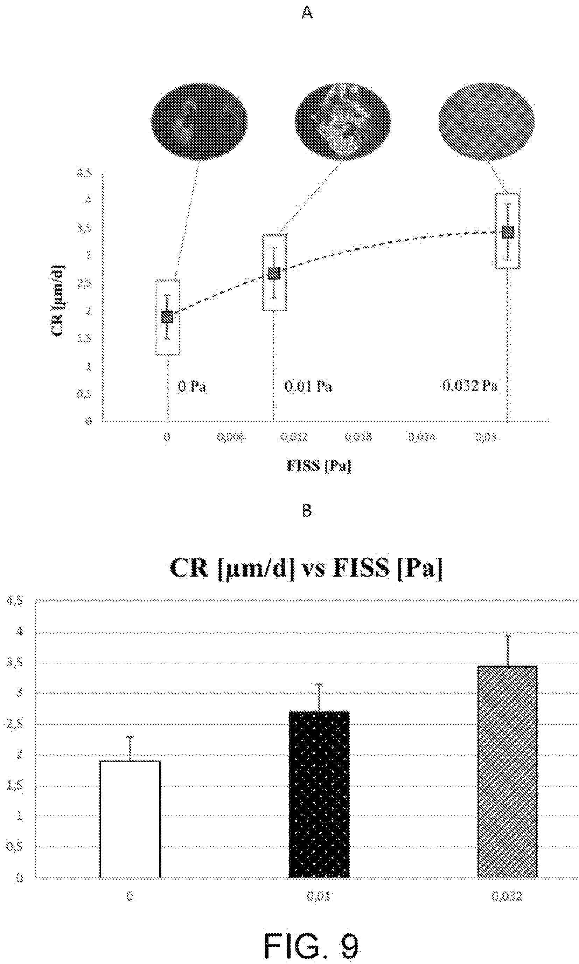

For the characterization of the material, uniform controlled corrosion was used on samples of pure magnesium. Confocal laser scanning microscopy (CLSM) data allowed evaluating the local decrease in sample heights and the corresponding corrosion rates in an in-vitro ocular environment. The imaging results were extended from a qualitative analysis to a local and quantitative analysis of corrosion rates by developing specific algorithms for post-image processing. A comparison between static and dynamic conditions was performed in terms of (i) decrease of the height of the profile, (ii) global analysis of the corrosion rate and evaluation of the volume loss. Finally, the morphologies of free eroded surfaces were acquired using a field emission scanning electron microscope. The key role that fluid dynamics has on magnesium corrosion has been correctly reported and correlated with the fluid movement (Efird, 2011), underlining the effects of shear stress on the fluid flow peculiar to the ocular system to magnesium samples manufactured ad hoc. Several surface morphologies were analyzed after different FISS stimulation (Figure 5), which included holes localizations, magnesium oxide (MgO) corrosion products and flat uniform corrosion; the presence of localizations and types of corrosion different from the uniform one (i.e. pitting corrosion) is strongly influenced by the morphology of the samples before the tests (Figure 5). The data show that the only areas where there is no uniformity of thickness are those where there were already localizations due to manufacturing defects. However, the influence of local fluid flow conditions is not so deep in the FISS reference range: in all three cases, fluid flow cleaned the surfaces from oxidized corrosion products, improving the uniform and flat erosion mechanism. This effect is more evident in the higher FISS fields: more serious localized attacks are only found in the upper FISS range, perhaps due to the fact that, after a certain threshold, the increase of FISS along the flow direction accelerated corrosion (Wang, 2014). The main importance of the uniform contribution in the corrosion mechanism was confirmed by the CLSM data acquired at different time scales during experimental biodegradation. In fact, the CLSM data of the sample profiles were post-processed and a normal distribution of the local height decrease was noted, for all the FISS configurations tested (Figure 6). Of course, the higher the FISS range the higher the decrease in height and the volume loss, reaching a decrease of 2.85 microns after a day of erosion (FISS = 0.032 Pa, figures 7-8). By analyzing the FISS values of 0.032 Pa, 0.01 Pa and 0 Pa, the corresponding corrosion velocity values were 3.4, 2.7 and 1.9 um/day, respectively (Figure 9). Thus, the most stressful ocular dynamic condition (FISS = 0.032 Pa) was used and coupled to the current clinical gap between two consecutive injections (one month), resulting in a potential pure magnesium thickness of 103 um. By changing the reference FISS range (0.01 Pa and 0 Pa), a thinner shell is needed, with thickness values of between 81 and 57 um.

Example 3: Evaluation of the release of active ingredients.

The release of active ingredients was analyzed with a reliable model capable of simulating the corrosion of a layer and thus the release of a single dose of drug encapsulated in the device. In this regard, a FEM modeling was developed capable of accurately recreating the flat and uniform magnesium corrosion in an ophthalmological environment. This allowed:

- defining the final shape and dimensions of the biodegradable layer;
- estimating the corrosion time from a known initial layer configuration;
- estimating the monthly corrosion rate of a biodegradable layer, its shape and size being known.

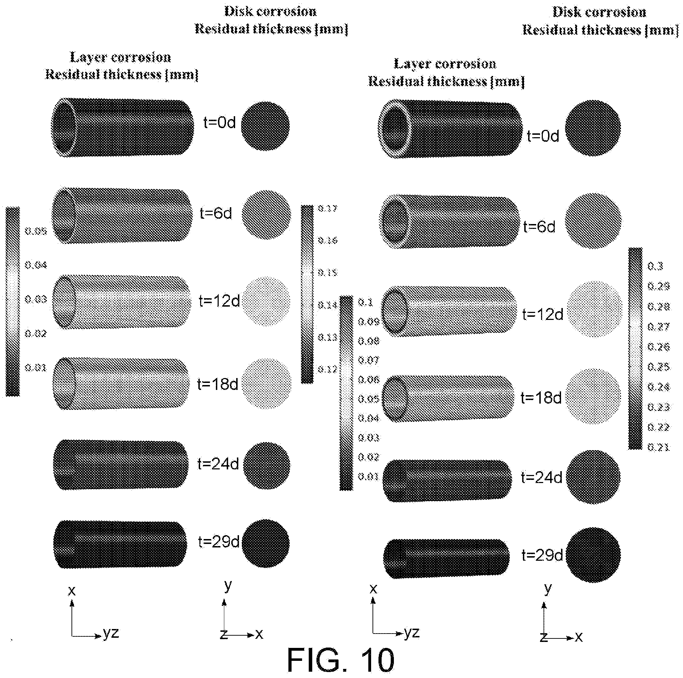

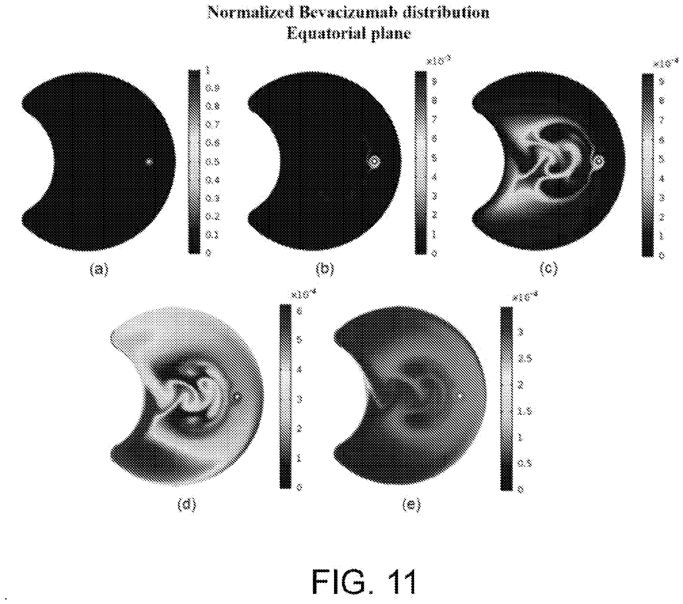

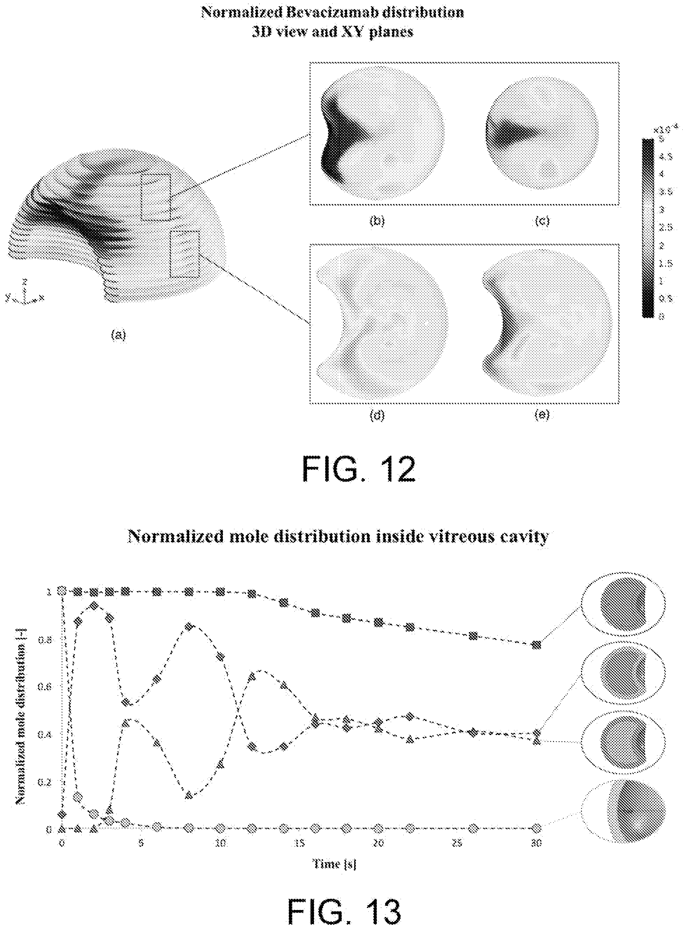

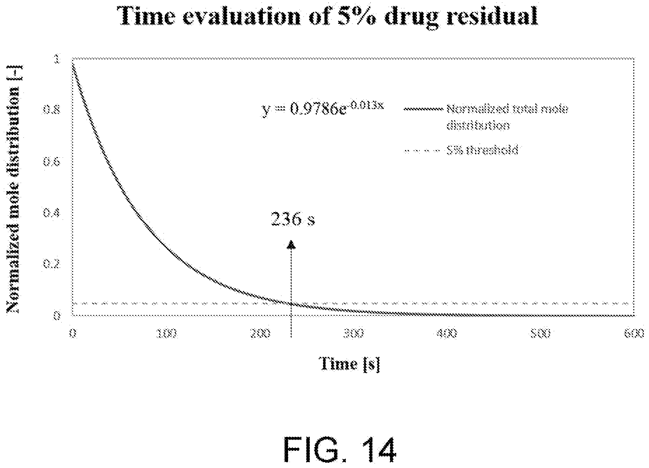

The proposed model, based on the finite element method (FEM), is capable of simulating more complex stress conditions (for example a non-homogeneous ocular shear stress field), which would require an optimization of the device configuration to support the complexity of corrosion phenomena and in any case allow a typical kinetic on-off release (Figure 10). The release model of bevacizumab from a monolayer device is indicative of the release mode of an encapsulated drug. The data obtained and shown in figures 11-13 show how convection plays a preponderant role with respect to diffusion. The time estimate for drug consumption (236 seconds) demonstrates the great influence of eye movements on drug delivery (Figure 14). The data obtained lead to the conclusion that, taking into account the most stressful configuration (FISS = 0.032 Pa), the layer of each wall of the solids constituting the device according to the present invention is, in a preferred embodiment, equal to 0.103 mm.

Example 4: Dissolution evaluation test.

The degradation rate of magnesium samples was tested using the imaging method described in example 1. The data obtained, shown in table 1 below, are indicative of the magnesium thicknesses required depending on the required degradation time, i.e. depending on the desired interval between the release of a first dose and the next dose.

Table 1

| Interval between the release of a dose and the next one (weeks) ⇒ | 2 | 3 | 4 | 6 | 8 | 12 | 16 | 20 | 24 |
|---|---|---|---|---|---|---|---|---|---|
| Degradation speed (μm/day) ⇓ | Shell thickness (μm) | | | | | | | | |
| 3.4 | 48 | 72 | 96 | 144 | 192 | 289 | 385 | 481 | 577 |
| 2.7 | 38 | 57 | 76 | 113 | 151 | 227 | 302 | 378 | 454 |
| 1.9 | 27 | 40 | 53 | 80 | 106 | 160 | 213 | 266 | 319 |

Advantageously, the device according to the present invention allows a scheduled and controlled administration of defined doses of drug over time, with a quantum release. In a preferred embodiment, where said device comprises one core and two crowns, with a single injection it is possible to schedule the administration of four doses of drug, where the first dose, not contained in the device, is administered simultaneously with the injection of the device, the next three are contained respectively in the first crown, in the second crown and in the core of the device, thus reducing the need for treatment from four injections to a single injection.

In an alternative embodiment, the device object of the present invention is applied as a subcutaneous implant, alternatively as an ingestible device, where the features of complete biodegradability and scheduled release of defined doses of active ingredient over time finds advantageous application also for subcutaneous implants and for ingestible devices.

The implantable device according to the present invention greatly reduces the impact of repeated invasive treatments. As an example, with a single injection it is possible to provide the anti-VEGF treatment required for at least three months in AMD. The costs of patient management and the risks of infection due to repeated injections decrease, patient compliance increases.

The use of magnesium as a biodegradable material, bio-inert material that generates bio-inert corrosion products, also overcomes the limits related to polymeric-based devices, where the same originate highly toxic corrosion products, such as to compromise the integrity and operation of the intraocular systems and generate a significant immune response.

## Claims

1. An implantable device for intraocular drug delivery comprising at least two hollow solids inscribed one into the other, and at least one closing element, where the walls of said at least two hollow solids are made of biodegradable material based on magnesium, and where said at least two hollow solids inscribed one into the other delimit a volume, called core, inside the innermost of said hollow solids and one or more volumes, called crown (s), comprised between said innermost hollow solid and the hollow solid at the exterior thereof, wherein said walls are impermeable continuous walls which become permeable as a function of the post-implantation time of said device, said hollow solids inscribed one into the other are arranged so as to have an upper base and/or a lower base in common, said core and said crowns (s) having at least one opening toward an outside, said at least one opening being said upper base and/or lower base; said core and said crown (s) being closed to the outside by said at least one closing element.

2. An implantable device according to claim 1, wherein in said core and/or in said crown (s) doses of at least one active ingredient are comprised.

3. A device according to claim 1 or 2, wherein said biodegradable material based on magnesium is an alloy selected from the group comprising alloys of aluminum - magnesium, lithium - magnesium, calcium - magnesium, zinc - magnesium, manganese - magnesium.

4. A device according to claim 3, wherein said alloy is a JDBM magnesium alloy: Mg-2.5Nd-0.2Zn-0.4Zr (wt%, JDBM).

5. A device according to any one of claims 2 to 4, wherein said active ingredient is selected from the group comprising anti-VEGF compounds, such as monoclonal antibodies: Ranibizumab, Bevacizumab, Brolucizumab; aptamers: Pegaptanib; fusion proteins: VEGF Trap Eye Aflibercept; Small-Interfering RNA (SiRNA); Anti-Platelet derived growth factor (PDGF); Anti Tyrosin Kinase or Tyrosine-kinase inhibitors; Anti receptor kinase; intracellular inhibitors of the tyrosine kinase cascade; kinase multi target inhibitors; mTOR inhibitors; integrin inhibitors; vascular disrupting agents (VGAs); anti-nicotine agents, anti-complement agents or complement inhibitors; immunomodulators; anti-oxidant agents; Ciliary neurotrophic factors (CNTF); corticosteroids; non-steroidal anti-inflammatory drugs; biological material, viral vectors including nucleotide sequences; cells, cells transfected with at least one of said viral vectors, individually or in combination with each other.

6. A device according to any one of claims 1 to 5, which is a cylindrical device (1) comprising three cylinders inscribed one into the other, which delimit three volumes within said device (1): a first crown (5), a second crown (6) and a core (7), wherein said first crown (5), said second crown (6) and said core (7) are each adapted to house a dose of drug.

7. A device according to claim 6, wherein the bases of said cylinders are concentric.

8. A device according to one of claims 1 to 5, which comprises three parallelepipeds inscribed one into the other which determine three volumes within said device (31): a first volume (35) which is a crown, a second volume (36) which is a crown, and a core (37), wherein said first volume (35), said second volume (36) and said core (37) are each adapted to house a dose of drug.

9. A method for the production of an implantable device which comprises:

- making a structure of magnesium and/or magnesium alloys, where said structure comprises at least two hollow solids inscribed one into the other defining at least one innermost volume, called core, and at least one volume called crown, where said hollow solids inscribed one into the other are arranged so as to have an upper base and/or a lower base in common, said core and said at least one volume called crown having at least one opening toward an outside, said at least one opening being said upper base and/or lower base;
- loading of said structure with one or more active ingredient;
- closing said at least one opening with a closing element.

**Patentansprüche**

1. Implantierbare Vorrichtung zur intraokularen Arzneimittelabgabe, die mindestens zwei ineinander verschachtelte Hohlfestkörper und mindestens ein Verschlusselement aufweist, wobei die Wände der mindestens zwei Hohlfestkörper aus biologisch abbaubarem Material auf Magnesiumbasis bestehen und wobei die mindestens zwei ineinander verschachtelte Hohlfestkörper ein Volumen, Kern genannt, innerhalb des innersten der Hohlfestkörper begrenzen und ein oder mehrere Volumina, Krone (Kronen) genannt, die zwischen dem innersten Hohlfestkörper und dem Hohlfestkörper an dessen Außenseite enthalten sind, wobei die Wände undurchlässige, durchgehende Wände sind, die durchlässig als Funktion der Zeit nach der Implantation der Vorrichtung werden, wobei die ineinander verschachtelten Hohlkörper so angeordnet sind, um eine gemeinsame obere Grundfläche und/oder eine gemeinsame untere Grundfläche zu haben, wobei der Kern und die Krone(n) mindestens eine Öffnung nach einer Außenseite haben, wobei die mindestens eine Öffnung die obere Grundfläche und/oder die untere Grundfläche ist; wobei der Kern und die Krone(n) zur Außenseite hin durch das mindestens eine Verschlusselement verschlossen sind.

2. Implantierbare Vorrichtung nach Anspruch 1, wobei im Kern und/oder in der Krone/den Kronen Dosierungen von mindestens einem Wirkstoff enthalten sind.

3. Vorrichtung nach Anspruch 1 oder 2, wobei das biologisch abbaubare Material auf Magnesiumbasis eine Legierung ist, die aus der Gruppe ausgewählt ist, die Legierungen aus Aluminium-Magnesium, Lithium-Magnesium, Kalzium-Magnesium, Zink-Magnesium, Mangan-Magnesium aufweist.

4. Vorrichtung nach Anspruch 3, wobei die Legierung eine JDBM-Magnesiumlegierung ist: Mg - 2,5 Nd - 0,2 Zn - 0,4 Zr (Gew.-%, JDBM).

5. Vorrichtung nach irgendeinem der Ansprüche 2 bis 4, wobei der Wirkstoff aus der Gruppe ausgewählt ist, die Anti-VEGF-Verbindungen, wie monoklonale Antikörper, aufweist: Ranibizumab, Bevacizumab, Brolucizumab; Aptamere: Pegaptanib; Fusionsproteine: VEGF Trap Eye Aflibercept; Small-Interfering RNA (SiRNA); Anti-Platelet-Derived Growth Factor (PDGF); Anti-Tyrosin-Kinase oder Tyrosin-Kinase-Hemmsubstanzen; Anti-Rezeptor-Kinase; intrazelluläre Hemmsubstanzen der Tyrosin-Kinase-Kaskade; Kinase-Mehrfachzielsetzungshemmsubstanzen; mTOR-Hemmsubstanzen; Integrin-Hemmsubstanzen; vaskuläre Störfaktoren (VGAs); Anti-Nikotin-Mittel, Anti-Komplement-Mittel oder Komplement-Hemmsubstanzen; Immunmodulatoren; Antioxidationsmittel; ziliare neurotrophische Faktoren (CNTF); Kortikosteroide; nicht-steroide entzündungshemmende Medikamente; biologisches Material, Virusüberträger einschließlich Nukleotidsequenzen; Zellen, Zellen transfiziert mit mindestens einem der Virusüberträgern, einzeln oder in Kombination miteinander.

6. Vorrichtung nach irgendeinem der Ansprüche 1 bis 5, die eine zylindrische Vorrichtung (1) mit drei ineinander verschachtelten Zylindern ist, die drei Volumina innerhalb der Vorrichtung (1) begrenzen: eine erste Krone (5), eine zweite Krone (6) und einen Kern (7), wobei die erste Krone (5), die zweite Krone (6) und der Kern (7) jeweils zur Aufnahme einer Arzneimitteldosierung ausgelegt sind.

7. Eine Vorrichtung nach Anspruch. 6, wobei die Grundflächen der Zylinder konzentrisch sind.

8. Vorrichtung nach irgendeinem der Ansprüche 1 bis 5, die drei ineinander verschachtelte Parallelepipede aufweist, die drei Volumen innerhalb der Vorrichtung (31) bestimmen: ein erstes Volumen (35), das eine Krone ist, ein zweites Volumen (36), das eine Krone ist und einen Kern (37), wobei das erste Volumen (35), das zweite Volumen (36) und der Kern (37) jeweils zur Aufnahme einer Arzneimitteldosierung ausgelegt sind.

9. Verfahren zur Herstellung einer implantierbaren Vorrichtung, das aufweist:

- Herstellen einer chemischen Zusammensetzung aus Magnesium und/oder Magnesiumlegierungen, wobei die chemischen Zusammensetzung mindestens zwei ineinander verschachtelte Hohlfestkörper aufweist, die mindestens ein innerstes Volumen, Kern genannt, und mindestens ein Volumen, Krone genannt, abgrenzen, wobei die ineinander verschachtelten Hohlfestkörper so angeordnet sind, um eine obere Grundfläche und/oder eine untere Grundfläche gemeinsam zu haben, wobei der Kern und das mindestens eine Volumen, Krone genannt, mindestens eine Öffnung zu einer Außenseite aufweisen, wobei die mindestens eine Öffnung die obere Grundfläche und/oder die untere Grundfläche ist;
- Beladen dieser chemischen Zusammensetzung mit einem oder mehreren Wirkstoffen;
- Schließen der mindestens einen Öffnung mit einem Verschlusselement.

## Revendications

1. Dispositif implantable pour l'administration d'un médicament intraoculaire comprenant au moins deux solides creux inscrits l'un dans l'autre, et au moins un élément de fermeture, où les parois desdits au moins deux solides creux sont constituées d'un matériau biodégradable à base de magnésium, et où lesdits au moins deux solides creux inscrits l'un dans l'autre délimitent un volume, dénommé noyau, à l'intérieur du plus interne desdits solides creux et un ou plusieurs volumes, dénommé(s) couronne(s), compris entre ledit solide creux le plus interne et le solide creux à l'extérieur de celui-ci, dans lequel lesdites parois sont des parois continues imperméables qui deviennent perméables au fur et à mesure de la durée écoulée après implantation dudit dispositif, lesdits solides creux inscrits l'un dans l'autre sont agencés de manière à avoir une base supérieure et/ou une base inférieure en commun, ledit noyau et ladite ou lesdites couronne(s)ayant au moins une ouverture sur un extérieur, ladite au moins une ouverture étant ladite base supérieure et/ou base inférieure ; ledit noyau et ladite ou lesdites couronne(s) étant fermés sur l'extérieur par ledit au moins un élément de fermeture.

2. Dispositif implantable selon la revendication 1, dans lequel dans ledit noyau et/ou ladite ou lesdites couronne(s) des doses d'au moins un principe actif sont comprises.

3. Dispositif selon la revendication 1 ou 2, dans lequel ledit matériau biodégradable à base de magnésium est un alliage sélectionné dans le groupe comprenant des alliages d'aluminium-magnésium, de lithium-magnésium, de calcium-magnésium, de zinc-magnésium, de manganèse-magnésium.

4. Dispositif selon la revendication 3, dans lequel ledit alliage est un alliage de magnésium JDBM : Mg-2.5Nd-0.2Zn-0.4Zr (% en poids, JDBM).

5. Dispositif selon l'une quelconque des revendications 2 à 4, dans lequel ledit principe actif est sélectionné dans le groupe comprenant des composés anti-VEGF, tels que des anticorps monoclonaux : ranibizumab, bévacizumab, brolucizumab ; des aptamères : pégaptanib ; des protéines de fusion : VEGF Trap Eye aflibercept ; des petits ARN interférents (siARN) ; un anti-facteur de croissance dérivé des plaquettes (PDGF) ; un anti-tyrosine kinase ou des inhibiteurs de la tyrosine-kinase ; un anti-récepteur kinase ; des inhibiteurs intracellulaires de la cascade des tyrosine kinases ; des inhibiteurs de tyrosine kinase multicibles ; des inhibiteurs de mTOR ; des inhibiteurs de l'intégrine ; des agents de destruction vasculaire (VGA) ; des agents anti-nicotine, des agents anti-complément ou des inhibiteurs du complément ; des immunomodulateurs ; des agents anti-oxydants ; des facteurs neurotrophiques ciliaires (CNTF) ; des corticoïdes ; des anti-inflammatoires non stéroïdiens ; un matériau biologique, des vecteurs viraux y compris des séquences nucléotidiques ; des cellules, des cellules transfectées avec au moins l'un desdits vecteurs viraux, individuellement ou en combinaison les uns avec les autres.

6. Dispositif selon l'une quelconque des revendications 1 à 5, qui est un dispositif cylindrique (1) comprenant trois cylindres inscrits les uns dans les autres, qui délimitent trois volumes à l'intérieur dudit dispositif (1) : une première couronne (5), une seconde couronne(6) et un noyau (7), dans lequel ladite première couronne(5), ladite seconde couronne(6) et ledit noyau (7) sont chacun adaptés pour renfermer une dose de médicament.

7. Dispositif selon la revendication 6, dans lequel les bases desdits cylindres sont concentriques.

8. Dispositif selon l'une quelconque des revendications 1 à 5, qui comprend trois parallélépipèdes inscrits les uns dans les autres qui déterminent trois volumes à l'intérieur dudit dispositif (31) : un premier volume (35) qui est une couronne, un second volume (36) qui est une couronne, et un noyau (37), dans lequel ledit premier volume (35), ledit second volume (36) et le noyau (37) sont chacun adaptés à renfermer une dose de médicament.

9. Procédé de production d'un dispositif implantable qui comprend :

- la réalisation d'une structure de magnésium et/ou d'alliage de magnésium, où ladite structure comprend au moins deux solides creux inscrits l'un dans l'autre définissant au moins un volume le plus interne, dénommé noyau, et au moins un volume dénommé couronne, où lesdits solides creux inscrits l'un dans l'autre sont agencés pour avoir une base supérieure et/ou une base inférieure en commun, ledit noyau et ledit au moins un volume dénommé couronne ayant au moins une ouverture sur un extérieur, ladite au moins une ouverture étant ladite base supérieure et/ou ladite base inférieure ;
- la charge de ladite structure avec un ou plusieurs principes actifs ;
- la fermeture de ladite au moins une ouverture avec un élément de fermeture.

FIG. 1

# FIG. 2

FIG. 3

(a)

(b)

(c)

FIG. 4

FIG. 5

FIG. 6

**FIG. 7**

**FIG. 8**

A

0 Pa  0.01 Pa  0.032 Pa

FISS [Pa]

B

## CR [μm/d] vs FISS [Pa]

# FIG. 9

FIG. 10

Normalized Bevacizumab distribution
Equatorial plane

(a)   (b)   (c)

(d)   (e)

FIG. 11

**Normalized Bevacizumab distribution
3D view and XY planes**

(b)

(c)

(d)

(e)

(a)

# FIG. 12

**Normalized mole distribution inside vitreous cavity**

Time [s]

# FIG. 13

**Time evaluation of 5% drug residual**

$y = 0.9786e^{-0.013x}$

——— Normalized total mole distribution

- - - - 5% threshold

236 s

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006110429 A
- WO 2011097634 A
- WO 2009097468 A
- US 2005244465 A
- US 2016045363 A
- US 2011054408 A

**Non-patent literature cited in the description**

- **LIU F et al.** *Mater Sci Eng C Mater Biol Appl.,* 2015, vol. 48, 400-7
- **STOCCHINO et al.** *Phys MedBiol,* 2007, vol. 52, 2021-2034
- **STOCCHINO et al.** *Phys Med Biol,* 2010, vol. 55, 453-67